# EUROPEAN PATENT APPLICATION

(11) **EP 3 689 361 A1**
(43) Date of publication of application: **05.08.2020**
(21) Application number: 18934523.4
(22) Date of filing: 27.12.2018
(51) Int. Cl.: A61K 36/45, A23L 33/105, A61P 3/10, A61P 9/10, A61P 13/12, A61P 1/16, A61P 25/28

(54) **APPLICATION OF CRANBERRY OR EXTRACT THEREOF IN PREPARATION OF MEDICATIONS OR HEALTH-CARE FOOD FOR TREATING, DELAYING OR ALLEVIATING AGES-RELATED DISEASES**

(30) Priority: 26.10.2018 CN 201811257604
(71) Applicant: BY-Health Co., Ltd., Guangdong 519040 (CN)
(72) Inventor: ZHANG, Xuguang., Guangdong 519040 (CN); HE, Ruikun., Guangdong 519040 (CN)
(74) Representative: Inspicos P/S
(86) International application number: PCT/CN2018/124288
(87) International publication number: WO 2020/082587

(57) **Abstract**

The present invention relates to the technical field of health foods, in particular the use of bilberry or its extract in the preparation of medicines or health foods for treating, delaying or alleviating AGEs-related diseases. The present invention verified through experiments that bilberry extract could inhibit the production of AGEs, thereby helping to delay and alleviate the occurrence and development of various diseases such as diabetes, atherosclerosis, chronic kidney disease, liver disease, and neurodegenerative diseases. The health foods formed by the combination of these compounds could provide a new idea and approach for preventing the occurrence and development of AGEs-related diseases.

## Description

The present application claims the priority of a Chinese patent application filed on October 26, 2018 with the Chinese Patent Office, with the application number 201811257604.9, and the title "an AGEs inhibitory composition and its use, preparation method and formulation", the disclosure of which is incorporated herein in its entirety by reference.

### Technical field

The present invention relates to the technical field of health foods, in particular to the use of bilberry or its extract in the preparation of medicines or health foods for treating, delaying or alleviating AGEs-related diseases.

### Background art

Advanced glycation end products (AGEs) refer to stable covalent adducts resulted from the spontaneous reaction of large molecules such as proteins, lipids, or nucleic acids with glucose or other reducing monosaccharides in the absence of enzymes. They are end products of non-enzymatic glycosylation reaction (Maillard reaction) and are excess sugar- and protein-bound products. Dozens of AGEs have been identified, such as carboxymethyllysine (CML), carboxyethyllysine (CEL), and pyrraline, and so on. Under pathological conditions, AGEs can be found in the corresponding diseased tissues.

There are two sources of AGEs in the body, one is that excess sugar and protein synthesize AGEs in the body, and the other is that AGEs present in food are taken into the body by eating. AGEs can combine with and destroy the tissue cells of the body. Under normal circumstances, AGEs in the body can be eliminated by kidneys. However, with increasing age, or under certain pathological conditions, AGEs accumulate in the body, which can have significant damage and other adverse effects on the body, interfere with normal physiological and biochemical processes in the body, affect the body's normal metabolism, and cause the occurrence and development of disease.

Current research proves that AGEs can accelerate the aging of human body and cause the occurrence of many chronic degenerative diseases, such as diabetes, atherosclerosis, chronic kidney disease, liver disease, and neurodegenerative diseases. Reducing AGEs can play a role of anti-aging and preventing various chronic degenerative diseases. Therefore, AGEs is also one of the hottest topics in the global medical community. Many scientific research institutions, universities, hospitals and pharmaceutical companies have joined the research of AGEs.

Aminoguanidine is currently the most studied AGEs inhibitor. Aminoguanidine is a small molecule nucleophilic hydrazine compound with antioxidant properties, which can eliminate hydroxyl radicals and cut off the cross-linking of AGEs, and can also lower the content of cholesterol and triacylglycerols, and reduce the accumulation of exogenous AGEs in the body. It mainly functions in two ways: ① one amino group of aminoguanidine can undergo addition reaction with a reducing carbonyl group in the Amadori product to form an inactive substance, thereby eliminating the carbonyl group, and preventing the Amadori product from further rearrangement; ② in the process of forming AGEs, the Amadori product needs the help of an active α-dicarbon-based intermediate, while the two amino groups at the ends of aminoguanidine can bind the active α-dicarbon-based intermediate to form a triazine compound, thereby blocking the change of the Amadori product into AGEs. However, aminoguanidine and its compounds were found to have adverse effects in clinical trials and could not be applied in clinical practice, so they have withdrawn from the stage of clinical research. Therefore, there is a need to develop novel AGEs inhibitors without adverse effects. At present, there is no report about bilberry or its extract as AGEs inhibitors.

### Contents of the invention

In view of this, the present invention provides the use of bilberry or its extract in the preparation of medicines or health foods for treating, delaying or alleviating AGEs-related diseases. The present invention verified through experiments that bilberry extract could inhibit the production of AGEs, thereby helping to delay and alleviate the occurrence and development of various diseases such as diabetes, atherosclerosis, chronic kidney disease, liver disease, and neurodegenerative diseases.

In order to achieve the above-mentioned object, the present invention provides the following technical solution:
The present invention provides the use of bilberry or its extract in the preparation of medicines or health foods for treating, delaying or alleviating AGEs-related diseases.

The mechanism of action of the present invention is explained as follows: Advanced glycation end products (AGEs) are highly active glycation end products formed by the glycation reaction (Maillard reaction) of amino groups of proteins, fatty acids, and nucleic acids with aldehyde groups of reducing sugars in the absence of enzymes in living organisms. This is a common phenomenon in living organisms. AGEs and their cross-linked products are stable and irreversible. They can activate a series of intracellular signaling pathways by acting on Receptor of Advanced glycation end products (RAGE) on the cell surface and start physiological and pathological reaction.

Under normal circumstances, AGEs in the body can be eliminated by kidneys. However, with increasing age, or under certain pathological conditions, AGEs accumulate in the body, which can have significant damage and other adverse effects on the body, interfere with normal physiological and biochemical processes in the body, affect the body's normal metabolism, and cause the occurrence and development of disease. Studies in recent years have shown that AGEs are involved in the occurrence and development of a variety of diseases, including diabetes, atherosclerosis, chronic kidney disease, liver disease, neurodegenerative diseases and the like. Therefore, it is necessary to find compounds or compositions that can lower the content of AGEs in human body, for protecting the body function, delaying or treating diseases, and achieving the goal of maintaining health.

Bilberry belongs to *Vaccinium* L of Ericaceae, including about 450 varieties. It is called as "*Vaccinium myrtillus*" in *Flora of China,* "*Vaccinium vitis-idaea*" in *International Business Standards* published by China Medical Insurance chamber of Commerce, and Bilberry in Taiwan, China. According to the No. 51 document of the Ministry of Health in 2002, bilberry has been included in the *List of Raw Materials for Health Foods.* Bilberry is a small deciduous shrub with flowering in June and fruit ripening in September, which often grows in patches. Bilberry is mainly distributed in northern and central Europe, and is also produced in Xinjiang, China.

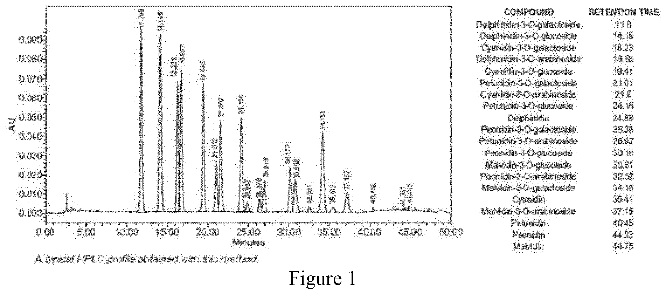

There are more than 15 cyanidins and about 1,000 anthocyanins in the plant kingdom, most of which are rare, and the anthocyanin content in plants is directly proportional to their antioxidant capacity. Bilberry is one of the fruits with the highest anthocyanin content. Its anthocyanin content exceeds that of cherries, raspberries, cranberries, blackberries, blueberries, blackcurrants, red orange and other fruits. It contains 0.3-0.7% of anthocyanins in fresh fruits, and the content increases with the maturity of the fruit. Bilberry has its unique anthocyanin composition (Figure 1). The chemical composition of its anthocyanins is different from that of all other fruits, including lowbush blueberry, highbush blueberry, *Vaccinium oxycoccus,* and the like. Clinical studies have proven that anthocyanins have antioxidant activity, vascular endothelial protective activity, anti-atherosclerotic activity, anti-tumor activity, immune stimulating effect, antiviral and antibacterial activity (flu, herpes), neuroplasticity and neuroprotective activity, retinal cell protective activity, and the like.

The processing of bilberry extract comprises firstly freezing freshly collected bilberry in a -35°C ventilation duct, and then subjecting it to various processing by machines: separating and obtaining individual fruits, removing small fruits, weighing, removing stones, washing and taking off leaves. Other red berries are removed from the bilberry by a system called Niagara. A conveyor belt transports all the fruits to a high platform and allows the fruits to fall freely. At this time, if a color sensor detects "different colors" in the fallen fruits, the fruits with "different colors" will be automatically removed. Finally, after manually picking and checking, the clean bilberry fruits are packed and ready for transport. The freeze-dried fruits are extracted with a water-ethanol solution to remove fruit residues, and a crude extract is obtained. After concentration and removal of insoluble impurities, the crude extract is sequentially subjected to resin adsorption, water washing, and ethanol washing processes to remove sugar-containing water, so that a pure extract is obtained. The pure extract is further concentrated and dried, and then packaged, which is a purified bilberry extract. The bilberry extract is consistent with the plant material in terms of the composition of anthocyanins, and various batches of extracts are also highly consistent in chemical composition with each other.

Preferably, the mass percentage of anthocyanins in the bilberry extract is 10% -50%.

Preferably, the mass percentage of anthocyanins in the bilberry extract is 10%-36%.

Preferably, the bilberry extract is an alcohol extract of bilberry fruits.

Preferably, the preparation method of bilberry extract comprises: freezing and pulverizing bilberry fruits, extracting with a water-ethanol solution and filtering to obtain a crude extract; after concentration and removal of insoluble impurities, subjecting the crude extract to resin adsorption, water washing, ethanol washing, concentrating and drying to obtain the bilberry extract.

In the present invention, the AGEs-related disease is diabetes, atherosclerosis, chronic kidney disease, liver disease, or neurodegenerative disease.

Preferably, the medicine further comprises a pharmaceutically acceptable excipient.

Preferably, the health food further comprises a food-acceptable excipient.

Preferably, the dosage form of the medicine or health food is an oral formulation or an injection formulation.

Preferably, the oral formulation is a tablet, a powder, a capsule, a granule, a pill, a pulvis, a cream, a solid drink or an oral liquid.

Preferably, the injection formulation is an injection solution or a powder for injection.

Preferably, in terms of parts by weight, the formula of the powder is:

| | |
|---|---|
| Bilberry extract | 50 ∼ 600 parts |
| Maltitol | 250 ∼ 750 parts |
| Water-soluble dietary fiber | 250 ∼ 750 parts |
| Maltodextrin | 250 ∼ 750 parts |
| Sweetener | 10 ∼ 50 parts |

Preferably, in terms of parts by weight, the formula of the tablet is:

| | |
|---|---|
| Bilberry extract | 50 ∼ 600 parts |
| Microcrystalline cellulose | 100 ∼ 300 parts |
| Carboxymethyl starch sodium | 10 ∼ 50 parts |
| Cross-linked povidone | 10 ∼ 50 parts |
| Magnesium stearate | 5 ∼ 20 parts |
| Silica | 5 ∼ 20 parts |
| Coating powder | 10 ∼ 50 parts |

Preferably, in terms of parts by weight, the formula of the hard capsule is:

| | |
|---|---|
| Bilberry extract | 50 ∼ 600 parts |
| Pre-gelatinized starch | 50 ∼ 200 parts |
| Magnesium stearate | 5 ∼ 20 parts |
| Silica | 5 ∼ 20 parts |

Preferably, in terms of parts by weight, the formula of the oral liquid is:

| | |
|---|---|
| Bilberry extract | 50 ∼ 600 parts |
| Purified water | 1000 ∼ 10000 parts |
| Oligosaccharide | 0 ∼ 100 parts |
| Sweetener | 10 ∼ 100 parts |

The oligosaccharide is a collective name of saccharides formed by connecting 2 to 10 monosaccharide molecules via glycosidic bonds, including, but not limited to, inulin, lactosucrose (LACT), xylooligosaccharide (XOS), isomaltooligosaccharide (IMO), galactooligosaccharide (GOS), fructooligosaccharide (FOS), soybean oligosaccharide (SOS), raffinose, stachyose and the like.

The sweetener includes, but not limited to, granulated sugar, honey, Momordica grosvenori, fructose, stevioside, sucralose, erythritol, xylitol, maltitol, mannitol (D-mannitol), sorbitol and the like.

The present invention provides the use of bilberry or its extract in the preparation of medicines or health foods for treating, delaying or alleviating AGEs-related diseases. The technical effects of the present invention are:
The present invention verified through experiments that bilberry extract could inhibit the production of AGEs, thereby helping to delay and alleviate the occurrence and development of various diseases such as diabetes, atherosclerosis, chronic kidney disease, liver disease, and neurodegenerative diseases. The health foods formed by the combination of these compounds could provide a new idea and approach for preventing the occurrence and development of AGEs-related diseases.

The test results of the present invention showed that plasma protein-bound AGEs in group A and group C were significantly reduced. Because most AGEs are protein-bound in vivo, AGEs are cross-linked with proteins, which causes them more difficult to degrade and thereby causing cumulative damage. Therefore, protein-bound AGEs are more representative of the cumulative and long-term exposure level of AGEs in the body. Bilberry extract with 36% anthocyanin content has the best effect on reducing AGEs.

The present invention provides an AGEs inhibitory composition which, in terms of parts by weight, consists of the following raw materials:
Bilberry extract 25 ∼ 500 parts, ginkgo biloba extract 15 ∼ 110 parts, green tea extract 25 ∼ 125 parts, rosemary extract 0.3 ∼ 20 parts, vitamin B₁ 0.1 ∼ 10 parts, vitamin B₂ 0.1 ∼ 10 parts, vitamin B₆ 0.1 ∼ 10 parts.

Preferably, in terms of parts by weight, the AGEs inhibitory composition consists of the following raw materials:
Bilberry extract 30 ∼ 150 parts, ginkgo biloba extract 20 ∼ 70 parts, green tea extract 40 ∼ 75 parts, rosemary extract 1 ∼ 20 parts, vitamin B₁ 0.5 ∼ 5 parts, vitamin B₂ 0.5 ∼ 5 parts, vitamin B₆ 0.5 ∼ 5 parts.

Preferably, in terms of parts by weight, the AGEs inhibitory composition consists of the following raw materials:
Bilberry extract 150 parts, ginkgo biloba extract 63 parts, green tea extract 75 parts, rosemary extract 5 parts, vitamin B₁ 5 parts, vitamin B₂ 5 parts, vitamin B₆ 5 parts.

In a specific embodiment provided by the present invention, in terms of parts by weight, the AGEs inhibitory composition consists of the following raw materials:
Bilberry extract 25 parts, ginkgo biloba extract 110 parts, green tea extract 25 parts, rosemary extract 0.3 parts, vitamin B₁ 0.1 parts, vitamin B₂ 0.1 parts, vitamin B₆ 10 parts.

In another specific embodiment provided by the present invention, in terms of parts by weight, the AGEs inhibitory composition consists of the following raw materials:
Bilberry extract 500 parts, ginkgo biloba extract 15 parts, green tea extract 125 parts, rosemary extract 20 parts, vitamin B₁ 10 parts, vitamin B₂ 10 parts, vitamin B₆ 0.1 parts.

In another specific embodiment provided by the present invention, in terms of parts by weight, the AGEs inhibitory composition consists of the following raw materials:
Bilberry extract 75 parts, ginkgo biloba extract 50 parts, green tea extract 60 parts, rosemary extract 20 parts, vitamin B₁ 2 parts, vitamin B₂ 2 parts, vitamin B₆ 2 parts.

In another specific embodiment provided by the present invention, in terms of parts by weight, the AGEs inhibitory composition consists of the following raw materials:
Bilberry extract 30 parts, ginkgo biloba extract 20 parts, green tea extract 40 parts, rosemary extract 1 part, vitamin B₁ 0.5 parts, vitamin B₂ 0.5 parts, vitamin B₆ 0.5 parts.

The present invention also provides the use of the AGEs inhibitory composition in the preparation of medicine or health food for treating, delaying or alleviating AGEs-related diseases.

In the present invention, the AGEs-related disease is diabetes, atherosclerosis, chronic kidney disease, liver disease, or neurodegenerative disease.

The present invention also provides a method for preparing the AGEs inhibitory composition, which comprises mixing various raw materials.

The present invention also provides an AGEs inhibitor, comprising the above-mentioned AGEs inhibitory composition and a health food acceptable excipient.

Preferably, the dosage form of the AGEs inhibitor is an oral formulation or an injection formulation.

Preferably, the oral formulation is a tablet, a powder, a capsule, a granule, a pill or an oral liquid.

Preferably, the injection formulation is an injection solution or a powder for injection.

The present invention provides an AGEs inhibitory composition and its use, preparation method and formulation. In terms of parts by weight, the AGEs inhibitory composition consists of the following raw materials: bilberry extract 25 ∼ 500 parts, ginkgo biloba extract 15 ∼ 110 parts, green tea extract 25 ∼ 125 parts, rosemary extract 0.3 ∼ 20 parts, vitamin B₁ 0.1 ∼ 10 parts, vitamin B₂ 0.1 ∼ 10 parts, vitamin B₆ 0.1 ∼ 10 parts. The technical effects of the present invention are:
The formula of the AGEs inhibitory composition of the present invention is reasonable, and the use of bilberry extract, ginkgo biloba extract, green tea extract, rosemary extract, vitamin B₁, vitamin B₂, and vitamin B₆ in combination can produce a significant synergistic effect in inhibiting AGEs production and degrading AGEs;

The AGEs inhibitory composition of the present invention can inhibit the production of AGEs and have a degrading effect on the produced AGEs, thereby helping to delay and alleviate the occurrence and development of various diseases such as diabetes, atherosclerosis, chronic kidney disease, liver disease, and neurodegenerative diseases. The health foods formed by the combination of these compounds can provide a new idea and approach for preventing the occurrence and development of AGEs-related diseases.

The AGEs inhibitory composition and its formulation can significantly reduce plasma protein-bound AGEs in the body.

### Brief description of the figures

Figure 1 shows the HPLC fingerprint of anthocyanins of bilberry.

### Detailed description of the invention

The present invention discloses the use of bilberry or its extract in the preparation of medicines or health foods for treating, delaying or alleviating AGEs-related diseases. Those skilled in the art can carry out the invention with a reference to the contents described herein by appropriately modifying the process parameters. In particular, it should be noted that all similar replacements and modifications will be apparent to those skilled in the art, and they are all considered to be included in the present invention. The method and use of the present invention have been described through preferred embodiments. It is obvious that the relevant persons can modify or appropriately alter and combine the method and use described herein without departing from the contents, spirit, and scope of the present invention, to realize and apply the technology of the present invention.

Bilberry extract: frozen bilberry fruits were pulverized, extracted with ethanol, subjected to two rounds of filtration, concentration and purification, spray-dried, sieved, packed, and tested.

The main functional ingredients of raw materials:

| Names of raw materials | Active ingredients | Content range | Detection method |
|---|---|---|---|
| Bilberry extract | anthocyanin | 10%-50% | Spectrophotometric/ HPLC |

Bilberry includes, but not limited to, *Vaccinium vitis-idaea, Vaccinnium vitis-idaea, Vaccinium uliginosum, Vaccinium virgatum (Vaccinium ashei;* Synonym V ashei), *Vaccinium angustifolium, Vaccinium australe, Vaccinium oxycoccus, Vaccinium macrocarpon, Vaccinium microcarpum, Vaccinium oxycoccus (Vaccinium oxycoccus,* small fruit cranberry, northern cranberry, cranberry*), Vaccinium erythrocarpum, Vaccinium crassifolium, Vaccinium ciliatum, Vaccinium oldhamii, Vaccinium delavayi, Vaccinium emarginatum, Vaccinium moupinense, Vaccinium nummularia, Vaccinium retusum, Vaccinium caesariense, Vaccinium hirsutum, Vaccinium koreanum, Vaccinium myrsinites* (evergreen blueberry), *Vaccinium myrtilloides* (Canadian blueberry), *Vaccinium pallidum* (dryland blueberry), *Vaccinium simulatum, Vaccinium tenellum, Vaccinium vacciniaceum, Vaccinium chunii, Vaccinium dunalianum, Vaccinium glaucoalbum (Vaccinium glauco-album), Vaccinium urceolatum, Vaccinium cylindraceum, Vaccinium hirtum, Vaccinium deliciosum, Vaccinium membranaceum, Vaccinium myrtillus, Vaccinium ovalifolium (V. alaskaense), Vaccinium parvifolium, Vaccinium praestans, Vaccinium ovatum* (California bilberry) and the like.

Ginkgo biloba extract: picking - pretreatment - extraction - concentration - extraction - spray drying - pulverization - sieving - final blending - inner packaging - outer packaging.

Green tea extract: green tea coarse powder is subjected to coarse crushing, extraction, concentration, filtration, spray drying and other processes.

Rosemary extract: rosemary leaves are subjected to pulverization, alcohol extraction, concentration, discoloration, column chromatography, spray drying and other processes.

The raw materials or auxiliary materials in the use of bilberry or its extract provided in the present invention in the preparation of medicines or health foods for treating, delaying or alleviating AGEs-related diseases are all available from the market.

The present invention is further described in combination with the following examples:

### Test example 1

### 1. Inhibitory effect of bilberry extracts with different anthocyanin contents on AGEs production

### Experimental principle:

Advanced glycation end products (AGEs) are highly active glycation end products formed by the glycation reaction (Maillard reaction) of amino groups of proteins, fatty acids, and nucleic acids with aldehyde groups of reducing sugars in the absence of enzymes in living organisms. This is a common phenomenon in living organisms. AGEs and their cross-linked products are stable and irreversible. They can activate a series of intracellular signaling pathways by acting on Receptor of Advanced glycation end products (RAGE) on the cell surface and start multiple physiological and pathological reactions.

Bovine serum albumin (BSA) can interact with Methylglyoxal (MGO) to produce AGEs. The compound to be tested was co-incubated with BSA and MGO, and the level of the AGEs production was determined through detecting change of fluorescence value in the system, to thereby evaluate whether or not the compound had inhibitory effect on the AGEs production. The positive control was aminoguanidine (AG), and the final concentration for initial screening was 100 µg / mL.

### Experimental results:

**Table 1. Inhibitory effect of bilberry extracts with different anthocyanin contents on AGEs production**

| No. | Compound name | AGEs production inhibition rate % Compound concentration 1mg/mL |
|---|---|---|
| 1 | Bilberry extract (36% anthocyanin) | 93.6 |
| 2 | Bilberry extract (25% anthocyanin) | 87.3 |
| 3 | Bilberry extract (10% anthocyanin) | 78.9 |
| Aminoguanidine | AG | 101.1 |

The results showed that the above bilberry extracts could inhibit the AGEs production, thereby helping to delay and alleviate the occurrence and development of various diseases such as diabetes, atherosclerosis, chronic kidney disease, liver disease, and neurodegenerative diseases. The health foods formed by the combination of these compounds could provide a new idea and approach for preventing the occurrence and development of AGEs-related diseases.

### 2. Evaluation on the AGEs inhibitory effect of bilberry extracts with different anthocyanin contents: randomized, double-blind, controlled trials

### Research objective:

In view that bilberry extract has potential anti-AGEs effect and its safety has been demonstrated by many clinical trials, we conducted randomized controlled trials to evaluate the antagonistic effect of bilberry extract against human AGEs.

Randomized controlled trials were conducted in healthy population. The levels of AGEs in human body were detected by UPLC-MS/MS before and after intervention, and the effect of bilberry extract on reducing AGEs was evaluated, which provided a new idea and population research evidence for reducing the exposure and harm of AGEs, and provided a research basis for the development of related health foods.

### Trial design:

The subjects were randomly grouped according to random number table into the following four groups: Group A (36% anthocyanin), Group B (10% anthocyanin), Group C (25% anthocyanin), and Group D (placebo group), and subjected to 3-month intervention trials.

### Intervention results:

(1) Diachronic changes of plasma in subjects suggested that, at the end of the intervention, Group A, Group B and Group C all had a significant decrease (P <0.05) in P_CML, P_CEL relative to the baseline; at the end of the intervention, Group D did not have a significant difference (P >0.05) in P_CML, P_CEL relative to the baseline. The results were shown in Tables 2 and 3.

**Table 2 Diachronic changes of plasma protein-bound carboxymethyllysine in various groups**

| Group | | Baseline | 1-month follow-up | 3-month follow-up | *P* |
|---|---|---|---|---|---|
| Group (µg/L) | *Median* | 1137.47 | 1209.96 | 840.91 | < 0.001 |
| | *Q1-Q3* | 918.45-1493.04 | 1001.67-1432.00 | 757.41-953.75 | |
| Group B (µg/L) | *Median* | 1105.02 | 1028.70 | 989.00 | 0.027 |
| | *Q1-Q3* | 959.22-1130.55 | 878.65-1076.90 | 896.01-1083.48 | |
| Group C (µg/L) | *Median* | 1124.47 | 1275.68 | 993.09 | < 0.001 |
| | *Q1-Q3* | 1021.19-1460.41 | 1030.02-1731.95 | 824.12-1052.31 | |
| Group D (µg/L) | *Median* | 1138.70 | 1117.02 | 1068.26 | 0.187 |
| | *Q1-Q3* | 1000.14-1327.33 | 994.94-1190.81 | 820.17-1156.60 | |

**Table 3 Diachronic changes of plasma protein-bound carboxyethyllysine in various groups**

| Group | | Baseline | 1-month follow-up | 3-month follow-up | *P* |
|---|---|---|---|---|---|
| Group | *Median* | 201.91 | 217.20 | 176.77 | 0.003 |
| A (µg/L) | *Q1-Q3* | 168.49-236.27 | 177.46-258.28 | 147.14-212.62 | |
| Group B (µg/L) | *Median* | 182.08 | 199.26 | 152.47 | 0.021 |
| | *Q1-Q3* | 152.62-215.40 | 190.43-212.42 | 113.54-214.27 | |
| Group C (µg/L) | *Median* | 260.36 | 211.15 | 200.21 | 0.019 |
| | *Q1-Q3* | 195.48-298.76 | 168.71-263.82 | 147.91-257.14 | |
| Group D (µg/L) | *Median* | 211.74 | 191.38 | 171.55 | 0.702 |
| | *Q1-Q3* | 176.25-269.80 | 176.36-219.13 | 142.77-223.29 | |

(2) Reader fluorescence detection of skin AGE
Fluorescence diachronic changes of skin AGEs in subjects suggested that, at the end of the intervention, Group A, Group B and Group C all showed a downward trend in skin AGEs with a significant difference (P <0.05) relative to the baseline; at the end of the intervention, Group D did not have a significant difference (P >0.05) in skin AGEs relative to the baseline. The results were shown in Table 4.

**Table 4 Diachronic changes of skin AGEs in various groups**

| Group | | Baseline | 1-month follow-up | 3-month follow-up | *P* |
|---|---|---|---|---|---|
| Group A | *Mean(Sd)* | 1.64(0.10) | 1.53(0.19) | 1.46(0.18) | <0.001 |
| Group B | *Mean(Sd)* | 1.63(0.23) | 1.52(0.29) | 1.40(0.24) | 0.007 |
| Group C | *Mean(Sd)* | 1.69(0.26) | 1.58(0.17) | 1.48(0.17) | <0.001 |
| Group D | *Mean(Sd)* | 1.63(0.25) | 1.56(0.18) | 1.50(0.17) | 0.056 |

### Summary:

At the end of the trials, plasma protein-bound AGEs were significantly reduced. Because most AGEs are protein-bound in vivo, AGEs are cross-linked with proteins, which causes them more difficult to degrade and thereby causing cumulative damage. Therefore, protein-bound AGEs are more representative of the cumulative and long-term exposure level of AGEs in the body.

Bilberry extract with 36% anthocyanin content had the best effect on reducing AGEs.

### Example 1 Powder (solid drink)

The formula was as follows:

**Table 5 Bilberry extract powder**

| Name of raw materials | Content range per serving |
|---|---|
| Bilberry extract | 50-600 |
| Maltitol | 250-750 |
| Water-soluble dietary fiber | 250-750 |
| Maltodextrin | 250-750 |
| Sweetener | 10-50 |

Preparation method: the bilberry extract was prepared into the powder according to conventional preparation process.

### Example 2 Tablet

The formula was as follows:

**Table 6 Bilberry extract tablet**

| Name of raw materials | Content range per serving |
|---|---|
| Bilberry extract | 50-600 |
| Microcrystalline cellulose | 100-300 |
| Carboxymethyl starch sodium | 10-50 |
| Cross-linked povidone | 10-50 |
| Magnesium stearate | 5-20 |
| Silica | 5-20 |
| Coating powder | 10-50 |

Preparation method: the bilberry extract was prepared into the tablet according to conventional preparation process.

### Example 3 Hard capsule

The formula was as follows:

**Table 7 Bilberry extract hard capsule**

| Name of raw materials | Content range per serving |
|---|---|
| Bilberry extract | 50-600 |
| Pre-gelatinized starch | 50-200 |
| Magnesium stearate | 5-20 |
| Silica | 5-20 |

Preparation method: the bilberry extract was prepared into the hard capsule according to conventional preparation process.

### Example 4 Oral liquid

The formula was as follows:

**Table 8 Bilberry extract oral liquid**

| Name of raw materials | Content range per serving |
|---|---|
| Bilberry extract | 50-600 |
| Purified water | 1000-10000 |
| Oligosaccharide | 0-100 |
| Sweetener | 10-100 |

Preparation method: the bilberry extract was prepared into the oral liquid according to conventional preparation process.

### Test example 2 Screening of AGE production inhibitors

### 1. Experimental principle:

Advanced glycation end products (AGEs) are highly active glycation end products formed by the glycation reaction (Maillard reaction) of amino groups of proteins, fatty acids, and nucleic acids with aldehyde groups of reducing sugars in the absence of enzymes in living organisms. This is a common phenomenon in living organisms. AGEs and their cross-linked products are stable and irreversible. They can activate a series of intracellular signaling pathways by acting on Receptor of Advanced glycation end products (RAGE) on the cell surface and start multiple physiological and pathological reactions.

Bovine serum albumin (BSA) can interact with Methylglyoxal (MGO) to produce AGEs. The compound to be tested was co-incubated with BSA and MGO, and the level of the AGEs production was determined through detecting change of fluorescence value in the system, to thereby evaluate whether or not the compound had inhibitory effect on the AGEs production. The positive control was aminoguanidine (AG), and the final concentration for initial screening was 100 µg / mL.

### 2. Experimental results:

**Table 9 Inhibitory effect of 7 samples on AGEs production**

| No. | Compound name | AGEs production inhibition rate % Compound concentration 1mg/mL |
|---|---|---|
| 1 | Bilberry extract | 93.6 |
| 2 | Green tea extract | 78.9 |
| 3 | Ginkgo biloba extract | 70.7 |
| 4 | Rosemary extract | 73.6 |
| 5 | Vitamin B₁ | 87.3 |
| 6 | Vitamin B₂ | 94.3 |
| 7 | Vitamin B₆ | 77.6 |
| Aminoguanidine | AG | 101.1 |

The results showed that the above 7 samples could inhibit the production of AGEs and have a degrading effect on the produced AGEs, thereby helping to delay and alleviate the occurrence and development of various diseases such as diabetes, atherosclerosis, chronic kidney disease, liver disease, and neurodegenerative diseases. The health foods formed by the combination of these compounds could provide a new idea and approach for preventing the occurrence and development of AGEs-related diseases.

### Example 5

The formula of this example was as follows:

**Table 10 Formula of AGEs inhibitory composition**

| Name of raw materials | Content (parts by weight) |
|---|---|
| Bilberry extract | 150 |
| Ginkgo biloba extract | 63 |
| Green tea extract | 75 |
| Rosemary extract | 5 |
| Vitamin B₁ | 5 |
| Vitamin B₂ | 5 |
| Vitamin B₆ | 5 |

Preparation method: various raw materials were weighed and mixed.

### Example 6

The formula of this example was as follows:

**Table 11 Formula of AGEs inhibitory composition**

| Name of raw materials | Content (parts by weight) |
|---|---|
| Bilberry extract | 25 |
| Ginkgo biloba extract | 110 |
| Green tea extract | 25 |
| Rosemary extract | 0.3 |
| Vitamin B₁ | 0.1 |
| Vitamin B₂ | 0.1 |
| Vitamin B₆ | 10 |

Preparation method: various raw materials were weighed and mixed.

### Example 7

The formula of this example was as follows:

**Table 12 Formula of AGEs inhibitory composition**

| Name of raw materials | Content (parts by weight) |
|---|---|
| Bilberry extract | 500 |
| Ginkgo biloba extract | 15 |
| Green tea extract | 125 |
| Rosemary extract | 20 |
| Vitamin B₁ | 10 |
| Vitamin B₂ | 10 |
| Vitamin B₆ | 0.1 |

Preparation method: various raw materials were weighed and mixed.

### Test Example 3 Inhibitory effect of compositions on AGEs production

With reference to the method of Test Example 1, the inhibitory effect of the AGEs inhibitory compositions of Examples 1-3 on AGEs production was examined. The test results were as follows:

**Table 13 Inhibitory effect of the AGEs inhibitory compositions on AGEs production**

| No. | Composition name | AGEs production inhibition rate % Composition concentration 1mg/mL |
|---|---|---|
| 1 | Composition of Example 1 | 96.6 |
| 2 | Composition of Example 2 | 94.7 |
| 3 | Composition of Example 3 | 95.1 |

The results showed that the compositions of Examples 1-3 could inhibit the production of AGEs and have a degrading effect on the produced AGEs, with the effect better than that of a single compound, thereby helping to delay and alleviate the occurrence and development of various diseases such as diabetes, atherosclerosis, chronic kidney disease, liver disease, and neurodegenerative diseases.

### Example 8

The formula of this example was as follows:

**Table 14 Formula of AGEs inhibitor**

| Raw materials | Tablet | Powder | Oral liquid |
|---|---|---|---|
| Bilberry extract | 150mg | 75mg | 30mg |
| Ginkgo biloba extract | 63mg | 50mg | 20mg |
| green tea extract | 75mg | 60mg | 40mg |
| Vitamin B₁ | 5mg | 2mg | 0.5mg |
| Vitamin B₂ | 5mg | 2mg | 0.5mg |
| Vitamin B₆ | 5mg | 2mg | 0.5mg |
| Rosemary extract | 5mg | 20mg | 1mg |
| Microcrystalline cellulose | 234mg | / | / |
| Carboxymethyl starch sodium | 24mg | / | / |
| Cross-linked povidone | 24mg | / | / |
| Silica | 8mg | / | / |
| Magnesium stearate | 6mg | / | / |
| Water-soluble dietary fiber | / | 1000mg | / |
| Resistant dextrin | / | 500mg | / |
| Xylitol | / | 1000mg | / |
| Juice powder | / | 1000mg | / |
| Purified water | / | / | 5000mg |
| Fruit juice | / | / | 2000mg |

Test Example 4 Evaluation on the AGEs inhibitory effect of samples: randomized, double-blind, controlled trials
1. Research objective:
The effect of the formula on reducing AGEs in healthy population was preliminarily evaluated.
2. Trial design:
The subjects were randomly grouped according to random number table into intervention group and placebo group and subjected to 3-month intervention trials. The intervention group population took the tablet of Example 8 at a dosage of 650 mg/tablet, 4 tablets per day.
3. Intervention results
The plasma samples of the test population in the two groups after 3-month intervention were all detected by UPLC-MS/MS, and eight biochemical indicators were detected. It was found that there was a statistical difference (P = 0.004) in the protein-bound CML level between the plasmas of the intervention group population and the placebo group population, i.e., relative to the placebo group population [841.66 (755.53-871.55) µg/L], the intervention group population had a lower protein-bound CML level [742.76 (701.65-819.13) µg/L]. As regards bound CEL in the plasma, the level in the intervention group population [105.69 (95.27-118.16) µg/L] was lower than that in the placebo group population [113.34 (100.50-137.96) µg/L], with a significant difference (P = 0.007). There was no statistical difference as to other indicators. The specific test indicator results were shown in the following table:

**Table 15 Test indicator results of placebo group and intervention group after 3-month intervention**

| Group | Placebo group | Intervention group | *P*-value |
|---|---|---|---|
| Number of people (*n*) | 35 | 39 | / |
| Skin AGEs | 1.48 (0.17) | 1.26 (0.18) | 0.003 |
| Protein-bound CML (µg/L) | 841.66 (755.53-871.55) | 742.76 (701.65-819.13) | 0.004 |
| Protein-bound CEL (µg/L) | 113.34 (100.50-137.96) | 105.69 (95.27-118.16) | 0.007 |

At the end of the trials, plasma protein-bound AGEs were significantly reduced in the intervention group. Because most AGEs are protein-bound in vivo, AGEs are cross-linked with proteins, which causes them more difficult to degrade and thereby causing cumulative damage. Therefore, protein-bound AGEs are more representative of the cumulative and long-term exposure level of AGEs in the body.

The above are only preferred embodiments of the present invention. It should be noted that, for those of ordinary skilled in the art, without departing from the principle of the present invention, a number of improvements and modifications can be made. It should be regarded that these improvements and modifications are also within the protection scope of the present invention.

## Claims

1. Use of bilberry or its extract in the preparation of medicines or health foods for treating, delaying or alleviating AGEs-related diseases.

2. The use as claimed in claim 1, **characterized in that** the mass percentage of anthocyanins in the bilberry extract is 10% -50%.

3. The use as claimed in claim 1 or 2, **characterized in that** the mass percentage of anthocyanins in the bilberry extract is 10% - 36%.

4. The use as claimed in claim 1, **characterized in that** the bilberry extract is an alcohol extract of bilberry fruits.

5. The use as claimed in claim 1, **characterized in that** the preparation method of the bilberry extract comprises: freezing and pulverizing bilberry fruits, extracting with a water-ethanol solution and filtering to obtain a crude extract; after concentration and removal of insoluble impurities, subjecting the crude extract to resin adsorption, water washing, ethanol washing, concentrating and drying to obtain the bilberry extract.

6. The use as claimed in claim 1, **characterized in that** the AGEs-related disease is diabetes, atherosclerosis, chronic kidney disease, liver disease, or neurodegenerative disease.

7. The use as claimed in claim 1, **characterized in that** the medicine further comprises a pharmaceutically acceptable excipient, and the health food further comprises a food-acceptable excipient.

8. The use as claimed in claim 7, **characterized in that** the dosage form of the medicine or health food is an oral formulation or an injection formulation.

9. The use as claimed in claim 8, **characterized in that** the oral formulation is a tablet, a powder, a capsule, a granule, a pill, a pulvis, a cream, a solid drink or an oral liquid.

10. The use as claimed in claim 8, **characterized in that** the injection formulation is an injection solution or a powder for injection.
